# EUROPEAN PATENT SPECIFICATION

(11) **EP 3 090 774 B1**
(45) Date of publication and mention of the grant of the patent: **30.08.2017**
(21) Application number: 16168562.3
(22) Date of filing: 06.05.2016
(51) Int. Cl.: A61M 16/08

(54) **BI-LUMEN BREATHING CIRCUIT ASSEMBLY**
BI-LUMEN-ATMUNGSSCHALTUNGSANORDNUNG
ENSEMBLE DE CIRCUIT RESPIRATOIRE À DEUX LUMIÈRES

(30) Priority: 08.05.2015 WO PCT/CN2015/078560
(43) Date of publication of application: 09.11.2016
(73) Proprietor: Shenzhen Envisen Industry Co., Limited, Shenzhen Guangdong 518115 (CN)
(72) Inventor: Yufei, Lu, 518115 Guangdong (CN)
(74) Representative: McIlroy, Steven David

(56) References cited:
- EP-A1- 0 460 731
- US-A1- 2014 150 794
- US-A1- 2014 276 178

## Description

### Technical Field

The invention relates generally to medical equipment, and more particularly to a bi-lumen (double-lumen) breathing circuit assembly comprising breathing circuit conduit lengths and a connector.

### Background

At present, only ordinary single-lumen corrugated or flexible connector circuits are compatible with breathing circuit accessories such as water collection cups for collecting exhaled breath condensate. An example of a "water trap" for connection with flexible tubes is disclosed in EP0460731 A1. However, the semi-circular or other irregular cross-sectional tube shapes of bi-lumen anaesthesia breathing tubes are typically incompatible with the standard circular cross-section connectors such as those provided on water collection cups. Examples of such breathing tubes are disclosed in US2014/150794 A1 and US 2014/276178 A1. In practice, bi-lumen breathing circuits can make the patient more comfortable by adding moisture into the injected oxygen or anaesthetic gas. However, condensate retained within the circuit for a long time is prone to result in the breeding of bacteria, harming patients and increasing risk of occurrence of ventilator-associated pneumonia. Since bi-lumen breathing tubes are provided in standard lengths, there can be clinical situations where it is necessary to extend the overall length of the tubes. Accordingly, there is a need for an improved bi-lumen breathing circuit assembly which overcomes, or at least ameliorates the aforementioned problems whilst enhancing patient health and wellbeing and improving convenience and flexibility in a wide range of clinical scenarios.

### Summary

The invention is defined in the appended claim 1.

According to a first aspect of the present invention, there is provided a bi-lumen breathing circuit assembly including first and second breathing circuit conduit lengths each comprising: (i) a lengthwise extending inner septum for segregating inspiratory and expiratory lumens; and (ii) lumen opening pairs at their respective opposite distal ends; the assembly further comprising a connector comprising: (iii) a lengthwise extending connector septum for segregating inspiratory and expiratory connector passages; and (iv) a pair of connector openings at their respective opposite distal ends; wherein a concavity is provided at each distal end of the connecting septum for sealingly receiving an inner septum of each breathing circuit conduit length thus ensuring a contiguous passage between connected lengths of breathing circuit conduits; and wherein the inspiratory and/or the expiratory connector passages within the connector is/are divided into separate inflow and outflow passages; wherein an intermediate inflow opening is provided in the, or each, inflow passage, and an intermediate outflow opening is provided in the, or each, outflow passage; and an accessory (3) is fitted between the inflow and outflow passages (220).

It will be appreciated that in its simplest form, the invention provides a quick and simple means of connecting together two bi-lumen breathing circuit conduit lengths in order to extend their overall length in accordance with clinical needs.

Optionally, an interference fit is defined between the respective septa.

Optionally, each inner septum is engageable within its corresponding concavity in a push-fit manner.

It will be appreciated that an interference push fit requires the depth dimension of the septa to be equal to, or slightly more than, the corresponding depth dimension of the concavity.

It will be appreciated that one of the two alternative connector arrangements defined above can adopt various different shapes. By way of non-limiting example, the overall shape of the connector may be a "TT" shape, or the shape of the mathematical "Pi" symbol. The inflow and outflow passages which define the upper parts of the "TT" or "Pi" shapes may adopt an inclined "V" shape. This shape minimises flow resistance and provides a natural guidance into, for example, a water collection cup.

It will be appreciated that the other of the two alternative connector arrangements defined above can adopt various different shapes. By way of non-limiting example, the overall shape of the connector may be similar mathematical "+" symbol. In this arrangement, an accessory such as a condensate collector may be fitted to the intermediate openings at both the inspiratory and the expiratory connector passages.

Optionally, the - or each - connector passage within the connector is divided into separate inflow and outflow passages by at least one contiguous extension of its connecting septum.

Optionally, the accessory is selected from the group comprising: a condensate collector, a filter, a humidifier or an automatic doser.

Optionally, distal end walls proximate the connector openings are sealingly connectable with a lumen opening pair of a breathing circuit conduit length.

Optionally, internal surface portions on each conduit length proximate each lumen opening pair are provided with a textured, ribbed or grooved surface profile.

It will be appreciated that such an internal surface profile enhances grip between the respective connector and conduit walls proximate the respective lumen and connector openings. The overall connection between the connector and each conduit length is enhanced further by providing the latter with a tapered external surface which diminishes towards each distal end. In this way, the two halves of each connector above and below each concavity hinge and are compressible towards one another when the connector engages with a conduit length. A reliable connection may additionally, or alternatively, be achieved by one or more of a clamp, sealing ring, collet, buckle or an adhesive.

Optionally, one bi-lumen breathing circuit conduit length is connectable to apparatus at a patient side, and the other bi-lumen breathing circuit conduit length is indirectly or directly connectable to an anaesthetic gas outlet, and to a carbon dioxide sampling connector.

### Brief Description of the Drawings

Embodiments of the present invention will now be described, by way of example only, with reference to the accompanying drawings in which:
Fig. 1 is a side view of a bi-lumen breathing circuit assembly of the present invention connected to a water collection cup (condensate collector);
Fig. 2 illustrates a partial cross-sectional view of the bi-lumen breathing circuit assembly of Fig. 1; and Fig. 2a illustrates a cross-sectional view of a "Y" shaped adapter;
Fig. 3 is a side view of a bi-lumen breathing circuit assembly connected to two water collection cups;
Fig. 4 illustrates a partial cross-sectional view of the bi-lumen breathing circuit assembly of Fig. 3;
Fig. 5 is a side view of a bi-lumen breathing circuit assembly connected to a group of extension tubes and one water collection cup;
Fig. 6 illustrates a partial cross-sectional view of the bi-lumen breathing circuit assembly of Fig. 5;
Fig. 7 is a side view of a bi-lumen breathing circuit assembly connected to two groups of extension tubes and two water collection cups;
Fig. 8 illustrates a partial cross-sectional view of the bi-lumen breathing circuit assembly of Fig. 7;
Fig. 9 is a side view of a bi-lumen breathing circuit assembly with an integrally formed accessory;
Fig. 10 illustrates a partial cross-sectional view of the bi-lumen breathing circuit assembly of Fig. 9;
Fig. 11 is a side view of a bi-lumen breathing circuit assembly comprising two integrally formed accessories;
Fig. 12 illustrates a partial cross-sectional view of the bi-lumen breathing circuit assembly of Fig. 11;
[Fig. 13 is a side view of a bi-lumen breathing circuit assembly adopting an alternative configuration;
Fig. 14 illustrates a partial cross-sectional view of one part of the bilumen breathing circuit assembly of Fig. 13;
Fig. 15 illustrates a partial cross-sectional view of another part of the bilumen breathing circuit assembly of Fig. 13;
Fig. 16 is a side view of a bi-lumen breathing circuit assembly adopting a further alternative configuration;
Fig. 17 illustrates a partial cross-sectional view of one part of the bi-lumen breathing circuit assembly of Fig. 16;
Fig. 18 illustrates a partial cross-sectional view of another part of the bi-lumen breathing circuit assembly of Fig. 16;
Fig. 19 is a side view of a bi-lumen breathing circuit assembly connected to a filter;
Fig. 20 is a side view of a bilumen breathing circuit assembly connected to a humidifier;
Fig. 21 is a side view of a bilumen breathing circuit assembly connected to an automatic doser (drug dispenser).

### Detailed Description of the Invention

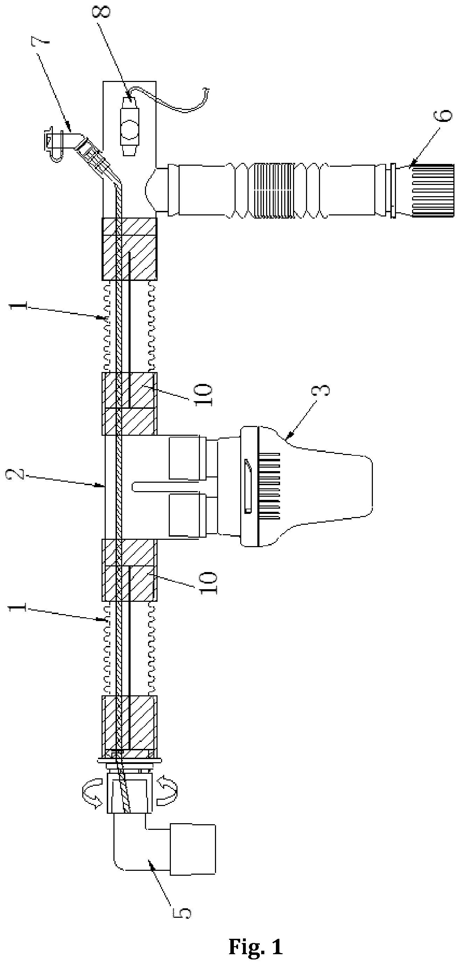

As shown in the figures, the present invention provides a bi-lumen breathing circuit assembly in which separate breathing circuit conduit lengths (1) can be joined together by differently arranged connectors or adapters (2), each having different shapes and associated accessories (3, 301, 302, 303) serving different functions. In each case, the breathing circuit conduit lengths (1) are provided with an inner septum (12) extending both diametrically and axially therein for segregating inspiratory and expiratory lumens (110). Each septum (12) terminates at the opposite distal ends (21) of the conduit lengths and forms a pair of semi-circular lumen openings. As shown in Fig. 1, one of the bi-lumen breathing circuits (1) is connected to patient-side connector (5), and the other bi-lumen breathing circuit 1 is connected to, or integrally moulded with, a gas collection plug (6) of an anaesthetic gas machine. The latter bi-lumen breathing circuit (1) is further connected to, or integrally moulded with, a carbon dioxide sampling connector (7) and a temperature probe and/or pressure sensor probe (8).

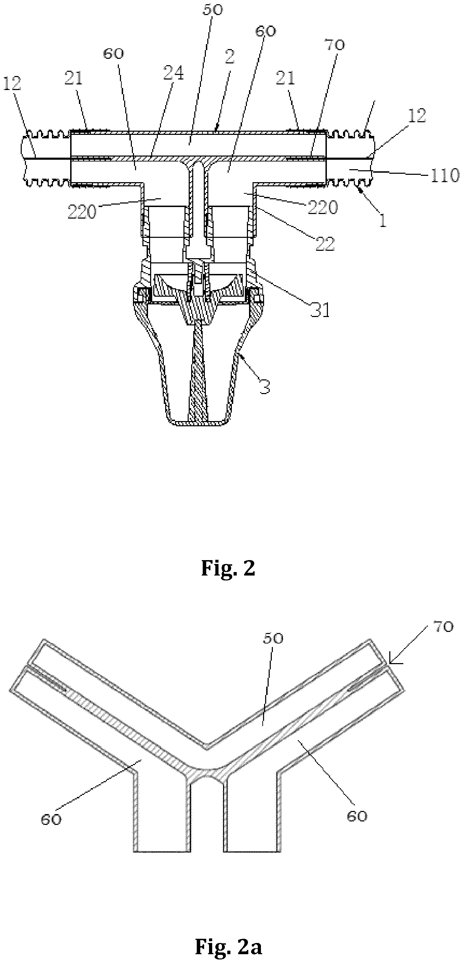

As shown in Fig. 2, the connector or adapter (2) adopts a "TT" or "Pi" shape. A lengthwise extending connector septum (24) segregates inspiratory and expiratory connector passages (50, 60) each of which terminate in connector openings at their respective opposite distal ends. An internal lengthwise concavity (70) in the form a slit is formed at each of the respective opposite distal ends of the connector septum (24). Each slit or concavity (70) is shaped and dimensioned to receive an inner septum (12) associated with a breathing circuit conduit length (1) in a sealing fashion thus ensuring good air-tightness and reliability.

The inner septum (12) and the slit or concavity (70) are shaped and dimensioned so as to provide an interference fit. Therefore, when a breathing circuit conduit length (1) is correctly orientated and pushed onto the connector or adapter (2) its inner septum (12) is forced into the slit or concavity (70) in a sealing manner and contiguous separated passages are provided between connected lengths of breathing circuit conduits (1) through the connector or adapter (2). Each conduit length (1) and its associated inner septum (12) is formed from an extruded plastics material and internal distal end surface portions thereof are provided with a textured, ribbed or grooved surface profile so as to enhance their grip on the external distal end surfaces of the connector or adapter (2) (also formed from a plastics material). The external distal end surfaces may be tapered in a manner in which their diameter reduces slightly towards their opposite distal ends. The tapered external end surfaces on the connector or adapter (2) enable the semi-circular lumen openings to be press-fitted thereon to effect a progressively tighter and more reliable seal between the connector or adapter (2) and the breathing circuit conduits (1) Alternatively, or additionally, a fastener (e.g. a sealing ring (10)) or an adhesive may be employed to effect a reliable seal.

Referring again to Fig. 2, the lower of the two connector passages (60) is divided into separate inflow and outflow passages (220), each of which are connected to a water collection cup (3) for collecting condensate. Condensate flows into the water collection cup (3) due to gravity. Removal of the condensate promotes patient health by preventing bacteria from breeding in the condensed water, thus reducing the occurrence of ventilator-associated pneumonia.

By way of example only, the lower left passage in Fig. 2 may be considered as the inflow passage (220) and the lower right passage may be considered as the outflow passage (220). An intermediate inflow opening is provided in the inflow passage and connects the inflow passage to the water collection cup (3). Similarly, an intermediate outflow opening connects the water collection cup (3) to the outflow passage at its distal end (22). Each connection may be effected in a similar press-fit manner to that described above in relation to the connection between the connector or adapter (2) and the breathing circuit conduits (1). This ensures good air tightness and reliability.

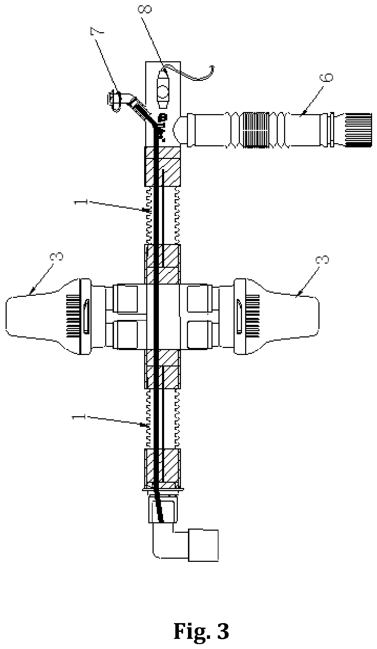

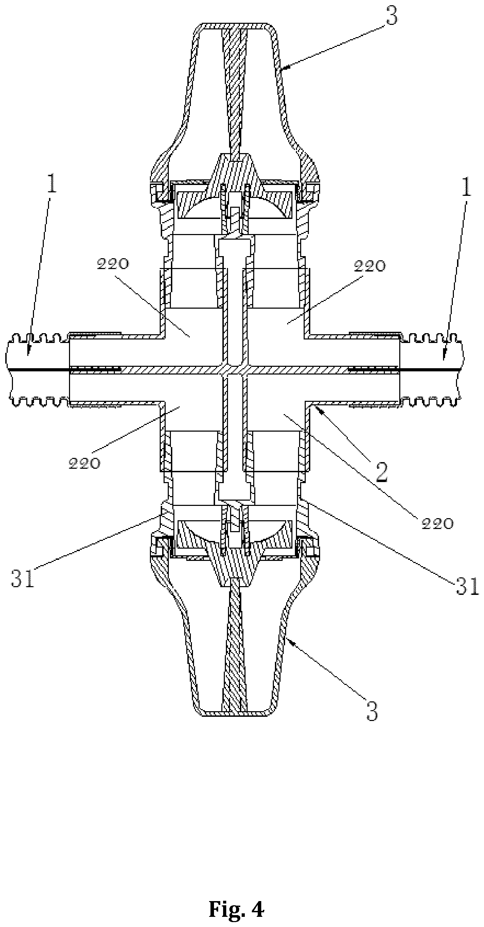

Referring now to the embodiment of Figs. 3 and 4, the connector or adapter (2) adopts a cross or "#" shape. The structure of the adapter is essentially the same as that described above in association with the embodiment of Figs 1 and 2. However, in this embodiment both the upper and the lower connector passages (50,60) is divided into separate inflow and outflow passages, each of which are connected to a separate water collection cups (3) via intermediate inflow and outflow openings.

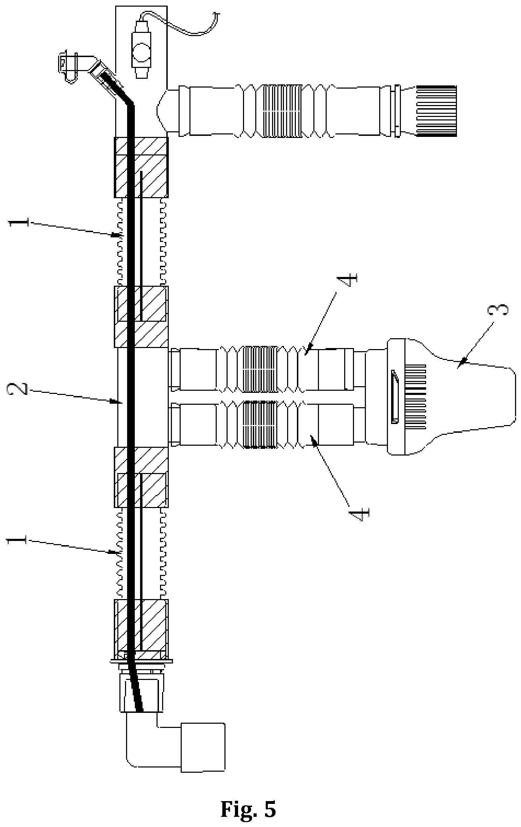

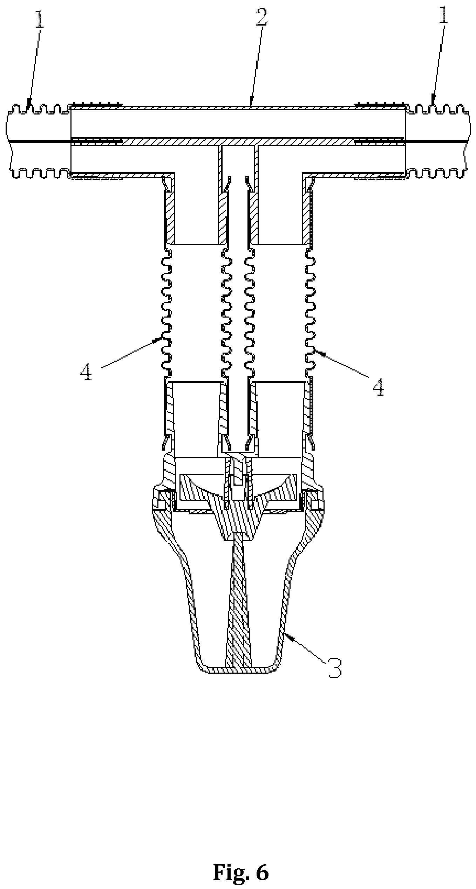

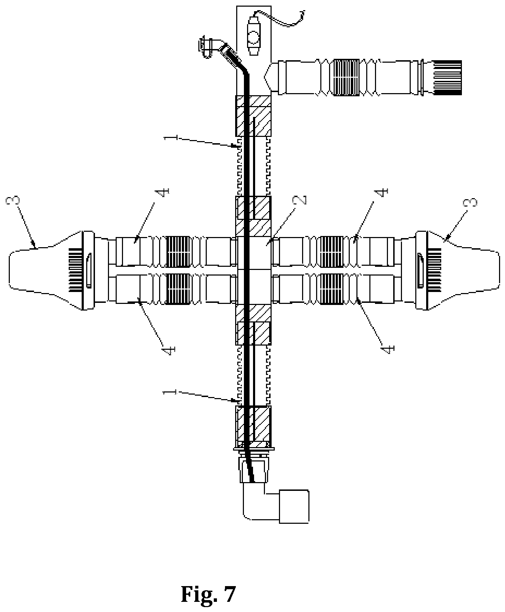

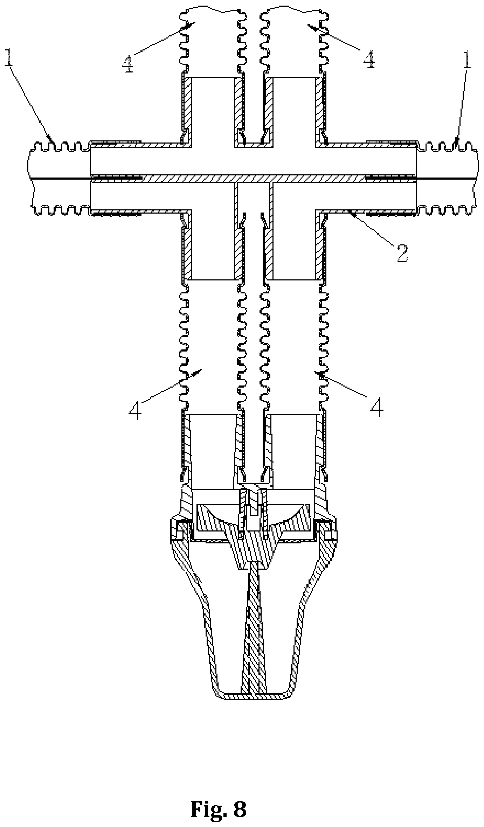

In the specific example illustrated in Fig. 2, intubation tubes (31) are inserted into to each of the intermediate inflow and outflow openings in a push-fit manner. The distal ends of the intermediate inflow and outflow openings may be provided with conical internal surface profiles to facilitate the connection. The opposite ends of the intubation tubes are inserted into the water collection cup (3). In the alternative embodiments of Figs. 5, 6 and 7, 8 extensions in the form of corrugated or flexible tubing connect the adapter (2) to the water collection cup (3).

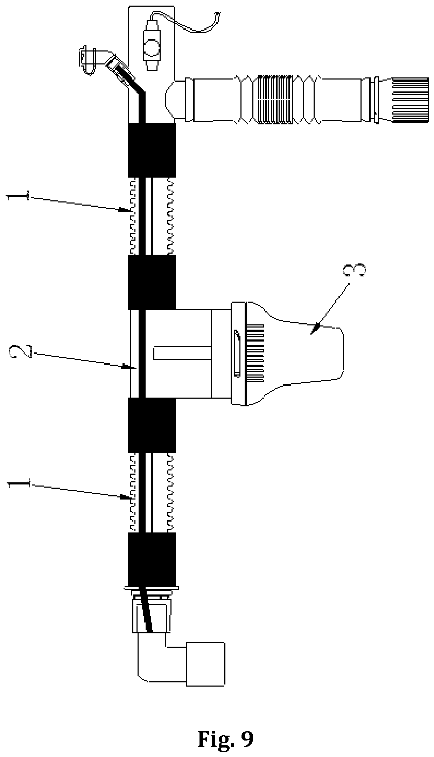

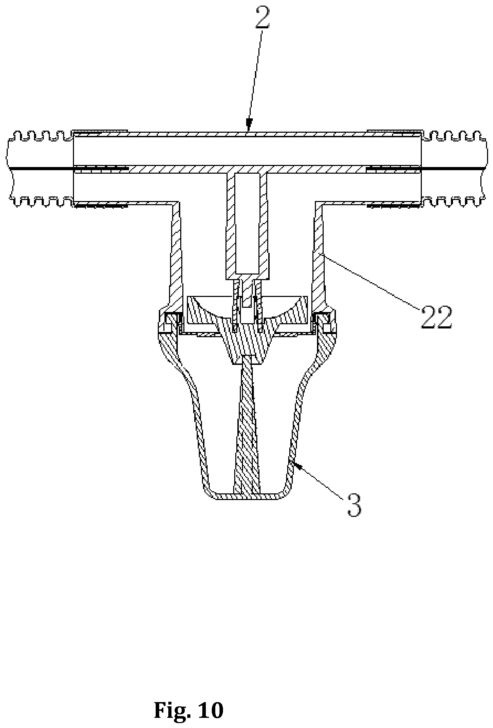

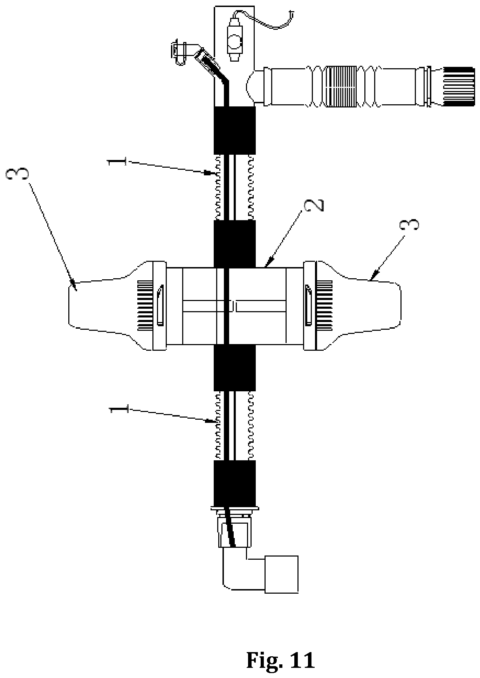

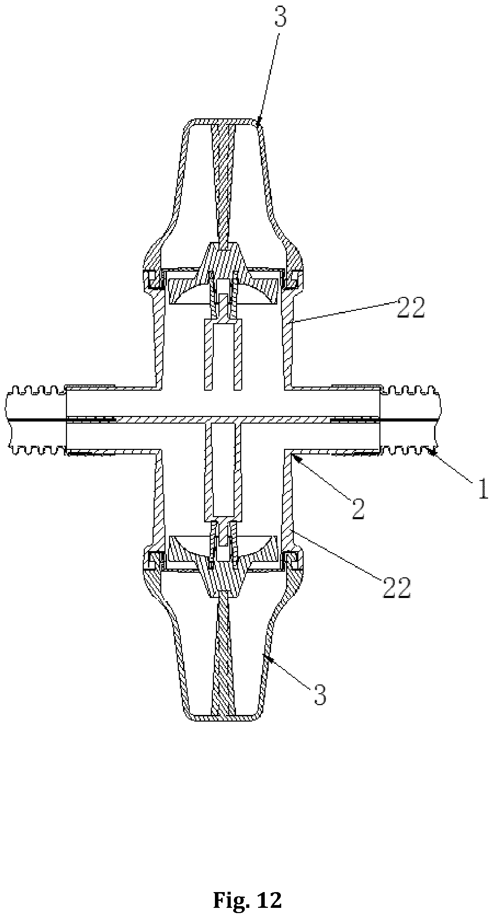

In the alternative embodiments of Figs. 9, 10 and 11, 12, the water collection cups (3) are provided as an integral part of the adapter (2) rather than as a separate connectable part.

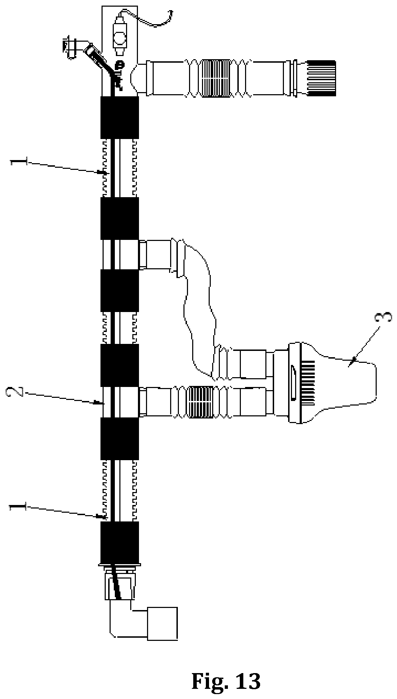

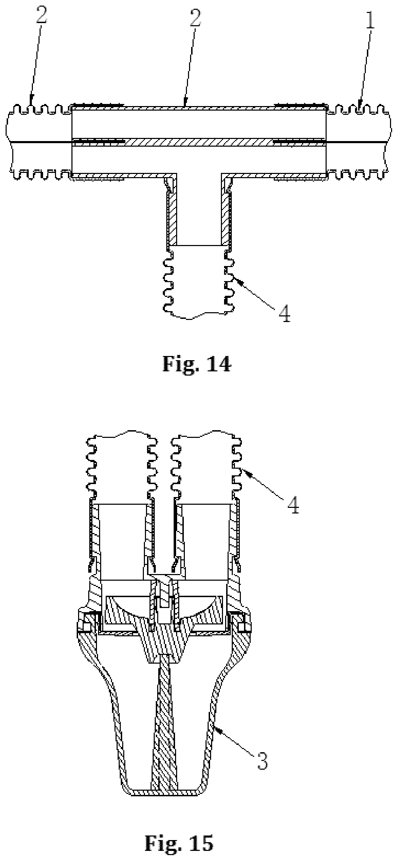

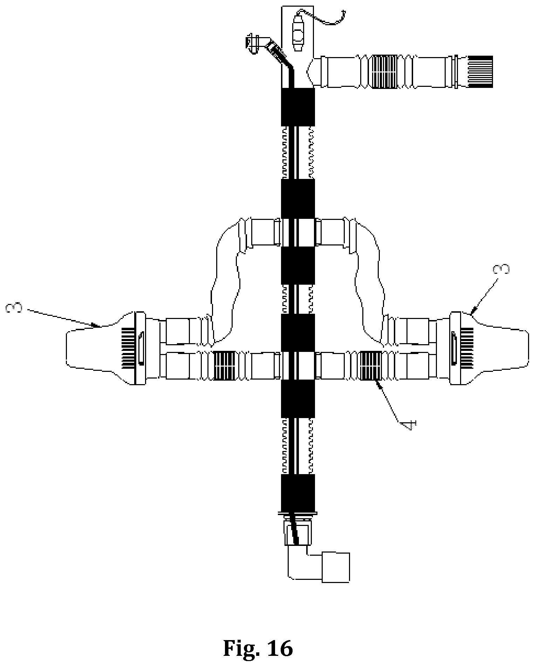

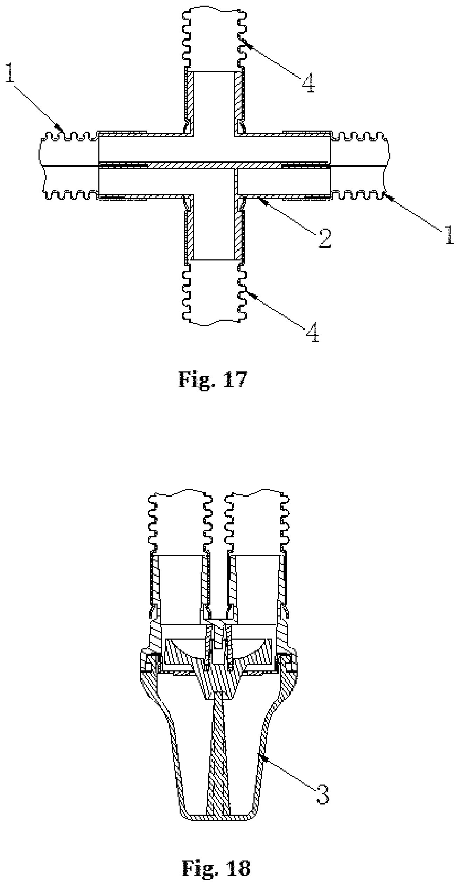

In the further alternative embodiments of Figs. 13, 14, 15 and 16, 17, 18, more complex combinations of adapters (2) and water collection cup (3) are illustrated which provide flexibility for a range of clinical scenarios.

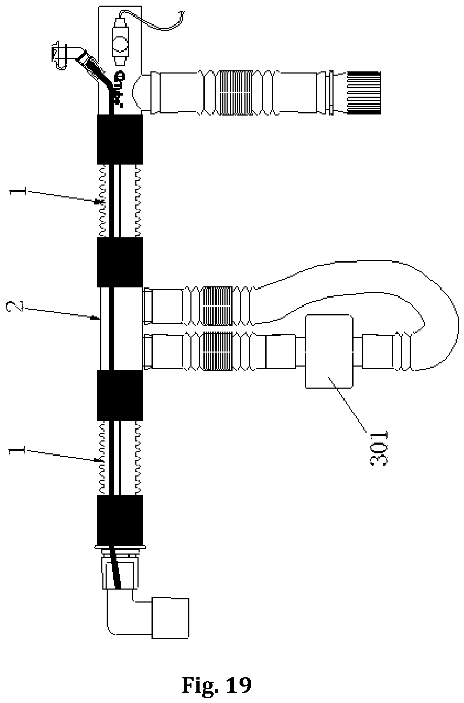

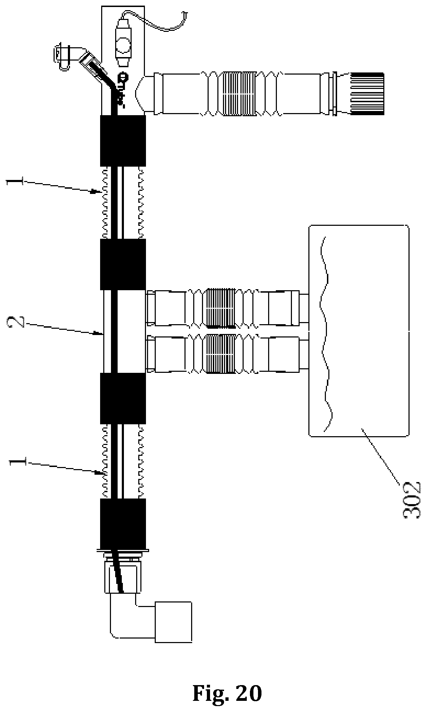

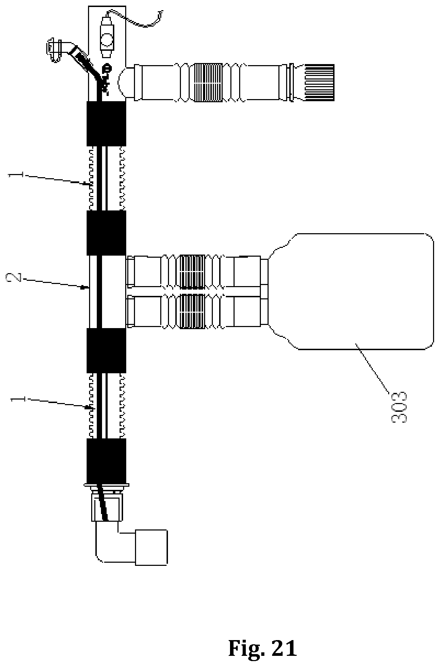

In further alternative embodiments alternative accessories are connected to the adapter (2), e.g. a filter (301) as shown in Fig. 19; a humidifier (302) as shown in Fig. 20; and an automatic drug dispenser (303) as shown in Fig. 21.

Although particular embodiments of the invention have been disclosed herein in detail, this has been done by way of example and for the purposes of illustration only. The aforementioned embodiments are not intended to be limiting with respect to the scope of the appended claims. Indeed, it is contemplated by the inventor that various substitutions, alterations, and modifications may be made to the invention without departing from the scope of the invention as defined by the accompanying claims.

## Claims

1. A bi-lumen breathing circuit assembly including first and second breathing circuit conduit lengths (1) each comprising:
(i) a lengthwise extending inner septum (12) for segregating inspiratory and expiratory lumens (110); and
(ii) lumen opening pairs at their respective opposite distal ends (21);
the assembly further comprising a connector (2) comprising:
(iii) a lengthwise extending connector septum (24) for segregating inspiratory and expiratory connector passages (50, 60); and
(iv) a pair of connector openings at its respective opposite distal ends;
wherein a concavity (70) is provided at each distal end of the connector septum (24) for sealingly receiving the inner septum (12) of each breathing circuit conduit length (1) thus ensuring a contiguous passage between connected breathing circuit conduit lengths; **characterised in that** the inspiratory (50) and/or the expiratory (60) connector passages within the connector (2) is/are divided into separate inflow and outflow passages (220); wherein an intermediate inflow opening is provided in the, or each, inflow passage, and an intermediate outflow opening is provided in the, or each, outflow passage; and an accessory (3) is fitted between the inflow and outflow passages (220).

2. A bi-lumen breathing circuit assembly according to claim 1, wherein an interference fit is defined between the respective septa (12, 24).

3. A bi-lumen breathing circuit assembly according to any of claims 1 or 2, wherein each inner septum (12) is engageable within its corresponding concavity (70) in a push-fit manner.

4. A bi-lumen breathing circuit assembly according to any preceding claim, wherein internal surface portions on each breathing circuit conduit length (1) are provided with a textured, ribbed or grooved surface profile.

5. A bi-lumen breathing circuit assembly according to any preceding claim, wherein the - or each - connector passage (50, 60) within the connector (2) is divided into separate inflow and outflow passages (220) by at least one contiguous extension of its connector septum (24).

6. A bi-lumen breathing circuit assembly according to any preceding claim , wherein the accessory (3) is selected from the group comprising: a condensate collector, a filter, a humidifier or an automatic doser.

7. A bi-lumen breathing circuit assembly according to any preceding claim, wherein distal end walls proximate the connector openings are sealingly connectable with a lumen opening pair of a breathing circuit conduit length (1).

8. A bi-lumen breathing circuit assembly according to any preceding claim, wherein internal surface portions on each breathing circuit conduit length (1) proximate each lumen opening pair are provided with a textured, ribbed or grooved surface profile.

9. A bi-lumen breathing circuit assembly according to any preceding claim, wherein one bi-lumen breathing circuit conduit length (1) is connectable to apparatus (5) at a patient side, and the other bi-lumen breathing circuit conduit length is indirectly or directly connectable to an anaesthetic gas outlet (6) and to a carbon dioxide sampling connector (7).

## Patentansprüche

1. Zweilumige Atmungskreislaufanordnung, aufweisend erste und zweite Atmungskreislaufleitungslängen (1), jeweils umfassend:
(i) ein längsweise sich erstreckendes inneres Septum (12) zur Trennung von Einatmungs- und Ausatmungslumen (110); und
(ii) Lumenöffnungspaare, an deren jeweilig entgegengesetzten distalen Enden (21);
wobei die Anordnung weiterhin einen Verbinder (2) umfasst, umfassend:
(iii) ein längsweise sich erstreckendes Verbinderseptum (24) zur Trennung von Einatmungs- und Ausatmungsverbinderdurchgängen (50, 60); und
(iv) ein Paar von Verbinderöffnungen an ihren jeweiligen entgegengesetzten distalen Enden;
wobei eine Konkavität (70) an jedem distalen Ende des Verbinderseptums (24) zum abdichtenden Aufnehmen des inneren Septums (12) von jeder Atmungskreislaufleitungslänge (1) bereitgestellt wird, wodurch ein zusammenhängender Durchgang zwischen verbundenen Atmungskreislaufleitungslängen gewährleistet ist; **dadurch gekennzeichnet, dass** die Einatmungs-Verbinderdurchgänge (50) und/oder die Ausatmungs-Verbinderdurchgänge (60) in dem Verbinder (2) in getrennte Einström- und Ausström-Durchgänge (220) aufgeteilt ist/sind; wobei eine Zwischeneinströmöffnung in dem oder jedem Einström-Durchgang bereitgestellt wird, und eine Zwischenausströmöffnung in dem oder jedem Ausström-Durchgang bereitgestellt wird; und ein Zusatzteil (3) zwischen den Einström- und Ausström-Durchgängen (220) eingerichtet ist.

2. Zweilumige Atmungskreislaufanordnung nach Anspruch 1, wobei eine Presspassung zwischen den jeweiligen Septa (12, 24) definiert ist.

3. Zweilumige Atmungskreislaufanordnung nach einem der Ansprüche 1 oder 2, wobei jedes innere Septum (12) in seiner entsprechenden Konkavität (70) in einer Steckweise eingrifffähig ist.

4. Zweilumige Atmungskreislaufanordnung nach einem der vorstehenden Ansprüche, wobei innere Oberflächenabschnitte an jeder Atmungskreislaufleitungslänge (1) mit einem texturierten, gerippten oder gerillten Oberflächenprofil ausgestattet sind.

5. Zweilumige Atmungskreislaufanordnung nach einem der vorstehenden Ansprüche, wobei der - oder jeder - Verbinderdurchgang (50, 60) in dem Verbinder (2) in getrennte Einström- und Ausström-Durchgänge (220) um mindestens eine zusammenhängende Ausdehnung von seinem Verbinderseptum (24) geteilt ist.

6. Zweilumige Atmungskreislaufanordnung nach einem der vorstehenden Ansprüche, wobei das Zusatzteil (3) ausgewählt ist aus der Gruppe, umfassend: einen Kondensatsammler, einen Filter, einen Befeuchter oder einen automatischen Dosierer.

7. Zweilumige Atmungskreislaufanordnung nach einem der vorstehenden Ansprüche, wobei distale Endwände nahe den Verbinderöffnungen mit einem Lumenöffnungspaar von einer Atmungskreislaufleitungslänge (1) abdichtend verbindbar sind.

8. Zweilumige Atmungskreislaufanordnung nach einem der vorstehenden Ansprüche, wobei innere Oberflächenabschnitte an jeder Atmungskreislaufleitungslänge (1) nahe jedem Lumenöffnungspaar mit einem texturierten, gerippten oder gerillten Oberflächenprofil ausgestattet sind.

9. Zweilumige Atmungskreislaufanordnung nach einem der vorstehenden Ansprüche, wobei eine zweilumige Atmungskreislaufleitungslänge (1) mit einer Einrichtung (5) an einer Patientenseite verbindbar ist, und die andere zweilumige Atmungskreislaufleitungslänge mit einem anästhetischen Gasauslass (6) und mit einem Kohlendioxid-Probenverbinder (7) indirekt oder direkt verbindbar ist.

## Revendications

1. Assemblage de circuit respiratoire à deux lumières incluant une première et une seconde longueur de conduit (1) de circuit respiratoire, chacune comprenant :
(i) une membrane interne (12) s'étendant en direction longitudinale pour la séparation des lumières inspiratoire et expiratoire (110) ; et
(ii) des paires d'ouvertures de lumières à leurs extrémités distales opposées respectives (21) ;
l'assemblage comprenant en outre un connecteur (2) comprenant :
(iii) une membrane de connexion (24) s'étendant en direction longitudinale pour la séparation des passages de connexion inspiratoire et expiratoire (50, 60) ; et
(iv) une paire d'ouvertures de connexion à ses extrémités distales opposées respectives ;
dans lequel une concavité (70) est prévue à chaque extrémité distale de la membrane de connexion (24) pour la réception en étanchéité de la membrane interne (12) de chaque longueur de conduit (1) du circuit respiratoire pour ainsi garantir un passage contigu entre les longueurs de conduits connectées du circuit respiratoire ;
**caractérisé en ce que** le(s) passage(s) de connexion inspiratoire (50) et/ou expiratoire (60) au sein du connecteur (2) est/sont divisé(s) en passages séparés d'entrée et de sortie (220) ;
dans lequel une ouverture d'entrée intermédiaire est prévue dans le passage d'entrée ou dans chacun de ceux-ci et une ouverture de sortie intermédiaire est prévue dans le passage de sortie ou dans chacun de ceux-ci ; et un accessoire (3) est inséré entre les passages d'entrée et de sortie (220).

2. Assemblage de circuit respiratoire à deux lumières selon la revendication 1, dans lequel un ajustement serré est défini entre les membranes respectives (12, 24).

3. Assemblage de circuit respiratoire à deux lumières selon l'une quelconque des revendications 1 ou 2, dans lequel chaque membrane interne (12) peut entrer en contact avec sa concavité correspondante (70) par emboîtement.

4. Assemblage de circuit respiratoire à deux lumières selon l'une quelconque des revendications précédentes, dans lequel des portions de surfaces internes sur chaque longueur de conduit (1) du circuit respiratoire sont munies d'un profil de surface texturé, nervuré ou rainuré.

5. Assemblage de circuit respiratoire à deux lumières selon l'une quelconque des revendications précédentes, dans lequel le passage de connexion (50, 60) ou chacun de ceux-ci au sein du connecteur (2) est divisé en passages séparés d'entrée et de sortie (220) par au moins un prolongement contigu de sa membrane de connexion (24).

6. Assemblage de circuit respiratoire à deux lumières selon l'une quelconque des revendications précédentes, dans lequel l'accessoire (3) est choisi parmi le groupe comprenant : un collecteur de condensat, un filtre, un humidificateur ou un doseur automatique.

7. Assemblage de circuit respiratoire à deux lumières selon l'une quelconque des revendications précédentes, dans lequel les parois terminales distales à proximité des ouvertures de connexion peuvent venir se connecter en étanchéité à une paire d'ouvertures de lumières d'une longueur de conduit (1) du circuit respiratoire.

8. Assemblage de circuit respiratoire à deux lumières selon l'une quelconque des revendications précédentes, dans lequel des portions de surfaces internes sur chaque longueur de conduit (1) du circuit respiratoire à proximité de chaque paire d'ouvertures de lumières sont munies d'un profil de surface texturé, nervuré ou rainuré.

9. Assemblage de circuit respiratoire à deux lumières selon l'une quelconque des revendications précédentes, dans lequel une longueur de conduit (1) du circuit respiratoire à deux lumières peut venir se connecter à un appareil (5) côté patient et l'autre longueur de conduit du circuit respiratoire à deux lumières peut venir se connecter de manière indirecte ou de manière directe à une sortie de gaz anesthésique (6) et à un connecteur d'échantillonnage de dioxyde de carbone (7).
